# FASCICULE DE BREVET EUROPEEN

(11) **EP 1 564 205 B1**
(45) Date de publication et mention de la délivrance du brevet: **14.05.2008**
(21) Numéro de dépôt: 05290310.1
(22) Date de dépôt: 11.02.2005
(51) Int. Cl.: C07C 253/30

(54) **Nouveau procédé de synthèse du (7-méthoxy-1-naphtyl) acétonitrile et application à la synthèse de l'agomélatine**
Verfahren zur Herstellung von (7-Methoxy-1-naphthyl) acetonitril und dessen Anwendung in der Synthese von Agomelatin
Process for synthesizing (7-methoxy-1-naphthyl) acetonitrile and its application in the synthesis of agomelatine

(30) Priorité: 13.02.2004 FR 0401437
(43) Date de publication de la demande: 17.08.2005
(73) Titulaire: Les Laboratoires Servier, 92415 Courbevoie Cedex (FR)
(72) Inventeur: Souvie, Jean-Claude, 76600 Le Havre (FR); Gonzalez Blanco, Isaac, 45002 Toledo (ES)

(56) Documents cités:
- EP-A- 0 447 285
- US-A- 3 931 188
- US-A- 3 992 403
- DEPREUX P ET AL: "SYNTHESIS AND STRUCTURE-ACTIVITY RELATIONSHIPS OF NOVEL NAPHTHALENIC AND BIOISOSTERIC AMIDIC DERIVATIVES AS MELATONIN RECEPTOR LIGANDS" JOURNAL OF MEDICINAL CHEMISTRY, AMERICAN CHEMICAL SOCIETY. WASHINGTON, US, vol. 37, no. 20, 30 septembre 1994 (1994-09-30), pages 3231-3239, XP002016146 ISSN: 0022-2623

## Description

La présente invention concerne un procédé de synthèse industriel du (7-méthoxy-1-naphtyl)acétonitrile, et son application à la production industrielle de l'agomélatine ou *N-*[2-(7-méthoxy-1-naphtyl)éthyl]acétamide.

Plus spécifiquement, la présente invention concerne un procédé de synthèse industriel du composé de formule (I) :

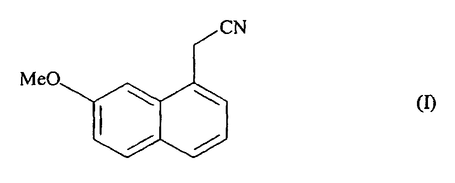

Le composé de formule (I) obtenu selon le procédé de l'invention est utile dans la synthèse de l'agomélatine ou *N*-[2-(7-méthoxy-1-naphtyl)éthyl]acétamide de formule (II) :

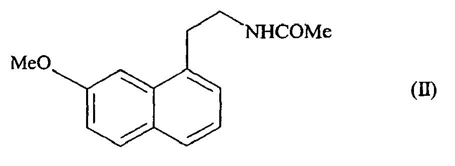

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone avec un rendement moyen inférieur à 30%.

Ce procédé implique l'action du bromoacétate d'éthyle, suivie d'une aromatisation et saponification pour conduire à l'acide correspondant, qui est ensuite transformé en acétamide puis déshydraté pour conduire au (7-méthoxy-1-naphtyl)acétonitrile, suivie d'une réduction puis de la condensation du chlorure d'acétyle.

En particulier, l'accès au (7-méthoxy-1-naphtyl)acétonitrile implique six étapes réactionnelles et, transposé à l'échelle industrielle, il a rapidement été mis en évidence des difficultés de mise en ceuvre de ce procédé dues principalement à des problèmes de reproductibilité de la première étape constituée par l'action du bromoacétate d'éthyle sur la 7-méthoxy-1-tétralone selon la réaction de Réformatsky conduisant au (7-méthoxy-3,4-dihydro-1(2*H*)-naphtalenylidène)éthanoate d'éthyle.
De plus, l'étape suivante d'aromatisation du (7-méthoxy-3,4-dihydro-1(2*H*)-naphtalenylidène)éthanoate d'éthyle était souvent partielle et conduisait, après saponification à un mélange de produits difficilement purifiable.

La littérature décrit l'accès en trois étapes au (7-méthoxy-1-naphtyl)acétonitrile à partir de la 7-méthoxy-1-tétralone par action de LiCH₂CN suivie d'une déshydrogénation au DDQ (2,3-dichloro-5,6-dicyano-1,4-benzoquinone) et enfin d'une déshydratation en milieu acide (Synthetic Communication, 2001, 31(4), 621-629). Toutefois le rendement global est moyen (76%) et surtout le DDQ utilisé dans la réaction de déshydrogénation ainsi que le reflux de benzène nécessaire à la troisième étape ne répondent pas aux contraintes industrielles de coût et d'environnement.

La demanderesse a présentement mis au point un nouveau procédé de synthèse industriel qui conduit, de façon reproductible et sans nécessiter de purification laborieuse, à l'agomélatine avec une pureté qui est compatible avec son utilisation comme principe actif pharmaceutique.

Une alternative aux difficultés rencontrées avec le procédé décrit dans le brevet EP 0 447 285 a été d'envisager l'aromatisation d'un substrat ne nécessitant pas de conditions drastiques et permettant l'utilisation de réactifs compatibles avec les exigences industrielles de coût et d'environnement.

La demanderesse a présentement mis au point un nouveau procédé de synthèse industrielle permettant d'obtenir le (7-méthoxy-1-naphtyl)acétonitrile de façon reproductible et sans nécessiter de purification laborieuse.

Plus spécifiquement, la présente invention concerne un procédé de synthèse industriel du composé de formule (I) : 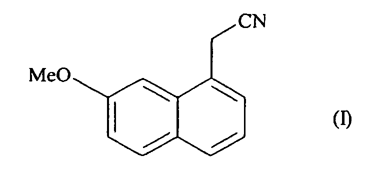 caractérisé en ce que l'on met en réaction le (7-méthoxy-3,4-dihydro-1-naphtalényl) acétonitrile (III) : 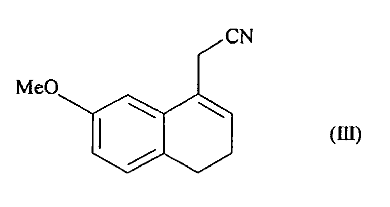 avec un catalyseur d'hydrogénation en présence d'un dérivé allylique,
pour conduire au composé de formule (I) après filtration et évaporation du solvant, composé de formule (I) que l'on isole sous la forme d'un solide après recristallisation,
étant entendu que :
par « dérivé allylique » on entend toute molécule contenant 3 à 10 atomes de carbone et pouvant contenir en plus 1 à 5 atomes d'oxygène, et contenant au moins un motif -CH₂-CH=CH₂.

De façon préférentielle, la réaction est réalisée au reflux du toluène ou du xylène et plus préférentiellement au reflux du toluène.

Le catalyseur utilisé préférentiellement est un catalyseur sous forme d'oxyde ou supporté comme par exemple le palladium, le platine, le nickel, Al₂O₃ et plus particulièrement le palladium. Avantageusement, on utilisera le palladium sur charbon, plus particulièrement le palladium sur charbon de 1 à 20% et encore plus particulièrement à 5% ou 10%. Préférentiellement, on utilisera du palladium sur charbon dans des quantités catalytiques, plus particulièrement dans des quantités allant de 1 à 10% en poids de catalyseur par rapport au poids de substrat et plus préférentiellement 5%.

Ce procédé est particulièrement intéressant pour les raisons suivantes :
- l'utilisation d'un catalyseur d'hydrogénation en présence d'un dérivé allylique et plus particulièrement du méthacrylate d'allyle est tout à fait compatible avec les exigences industrielles de coût et d'environnement, contrairement aux quinones couramment utilisées,
- il permet de plus d'obtenir à l'échelle industrielle le composé de formule (I) de façon exclusive, en particulier exempt du produit de réduction correspondant de formule (IV) : 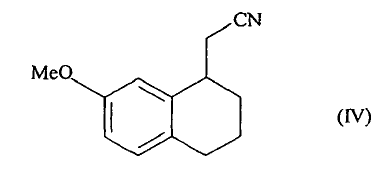
- enfin les taux de transformations observés sont élevés, supérieurs à 90%.

Le composé de formule (I) ainsi obtenu est, le cas échéant, soumis à une réduction puis à une réaction de couplage avec l'anhydride acétique pour conduire à l'agomélatine.

### Exemple 1 : (7-Méthoxy-1-naphtyl)acétonitrile

Dans un réacteur de 670 1 sont introduits 12,6 kg de palladium sur charbon à 5% dans du toluène et portés à reflux, puis 96,1 kg de (7-méthoxy-3,4-dihydro-1-naphtalényl) acétonitrile en solution dans du toluène sont ajoutés ainsi que 63,7 kg de méthacrylate d'allyle. La réaction se poursuit à reflux et est suivie par chromatographie en phase vapeur. Lorsque tout le substrat de départ a disparu, le milieu réactionnel est refroidi à l'ambiante puis filtré. Après évaporation du toluène, le résidu solide obtenu est recristallisé dans un mélange éthanol/eau (80/20) pour conduire au produit du titre avec un rendement de 91% et une pureté chimique supérieure à 99%.

*Point de fusion : 83°C*

## Revendications

1. Procédé de synthèse industriel du composé de formule (I) 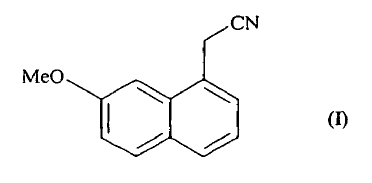 **caractérisé en ce que** l'on met en réaction le (7-méthoxy-3,4-dihydro-1-naphtalényl) acétonitrile (III) : 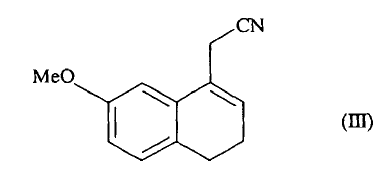 avec un catalyseur d'hydrogénation en présence d'un dérivé allylique,
pour conduire au composé de formule (I) après filtration et évaporation du solvant, composé de formule (I) que l'on isole sous la forme d'un solide après recristallisation.

2. Procédé de synthèse du composé de formule (I) selon la revendication 1 **caractérisé en ce que** la réaction est réalisée au reflux du toluène.

3. Procédé de synthèse du composé de formule (I) selon la revendication 1, **caractérisé en ce que** le catalyseur d'hydrogénation est le palladium.

4. Procédé de synthèse du composé de formule (I) selon la revendication 1, **caractérisé en ce que** le catalyseur d'hydrogénation est le palladium sur charbon à 5%.

5. Procédé de synthèse du composé de formule (I) selon la revendication 1, **caractérisé en ce que** la quantité de catalyseur d'hydrogénation utilisée est de 5% en poids de catalyseur par rapport au poids de substrat.

6. Procédé de synthèse de l'agomélatine à partir du composé de formule (I), **caractérisé en ce que** le composé de formule (I) est obtenu par le procédé de synthèse selon l'une quelconque des revendications 1 à 5, et que l'on soumet à une réduction puis à un couplage avec l'anhydride acétique.

## Claims

1. Process for the industrial synthesis of the compound of formula (I) 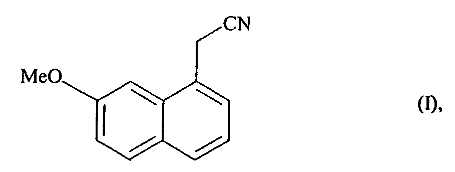 **characterised in that** (7-methoxy-3,4-dihydro-1-naphthalenyl)acetonitrile (III) : 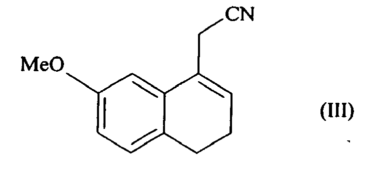 is reacted with a hydrogenation catalyst in the presence of an allyl compound
to yield the compound of formula (I) after filtration and evaporation of the solvent, which compound of formula (I) is isolated in the form of a solid after recrystallisation.

2. Process for the synthesis of the compound of formula (I) according to claim 1, **characterised in that** the reaction is carried out with reflux of toluene.

3. Process for the synthesis of the compound of formula (I) according to claim 1, **characterised in that** the hydrogenation catalyst is palladium.

4. Process for the synthesis of the compound of formula (I) according to claim 1, **characterised in that** the hydrogenation catalyst is 5% palladium-on-carbon.

5. Process for the synthesis of the compound of formula (I) according to claim 1, **characterised in that** the amount of hydrogenation catalyst used is 5% by weight of catalyst in relation to the weight of substrate.

6. Process for the synthesis of agomelatine starting from the compound of formula (I), **characterised in that** the compound of formula (I) is obtained by the synthesis process according to any one of claims 1 to 5, and is subjected to reduction and then to coupling with acetic anhydride.

## Patentansprüche

1. Verfahren zur technischen Synthese der Verbindung der Formel (I): 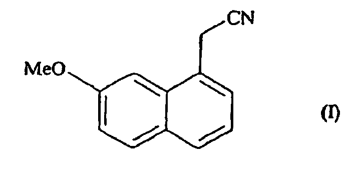 **dadurch gekennzeichnet, dass** man (7-Methoxy-3,4-dihydro-1-naphthalinyl)-acetonitril (III): 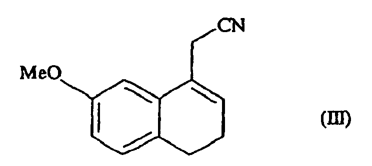 in Gegenwart eines Allylderivats mit einem Hydrierkatalysator umsetzt, sodass man nach der Filtration und dem Verdampfen des Lösungsmittels die Verbindung der Formel (I) erhält, welche man nach der Umkristallisation in Form eines Feststoffs erhält.

2. Verfahren zur Synthese der Verbindung der Formel (I) nach Anspruch 1, **dadurch gekennzeichnet, dass** die Reaktion am Rückfluss in Toluol durchgeführt wird.

3. Verfahren zur Synthese der Verbindung der Formel (I) nach Anspruch 1, **dadurch gekennzeichnet, dass** man als Hydrierkatalysator Palladium verwendet.

4. Verfahren zur Synthese der Verbindung der Formal (I) nach Anspruch 1, **dadurch gekennzeichnet, dass** man als Hydrierkatalysator 5 % Palladium-auf-Kohlenstoff verwendet.

5. Verfahren zur Synthese der Verbindung der Formel (I) nach Anspruch 1, **dadurch gekennzeichnet, dass** die verwendete Menge des Hydrierkatalysators 5 Gew.-% Katalysator, bezogen auf das Gewicht des Substrats, beträgt.

6. Verfahren zur Synthese von Agomelatin ausgehend von der Verbindung der Formel (I), **dadurch gekennzeichnet, dass** man die Verbindung der Formel (I) nach dem Syntheseverfahren nach einem der Ansprüche 1 bis 5 herstellt und sie einer Reduktion und dann einer Kupplung mit Essigsäureanhydrid unterwirft.
